# EUROPEAN PATENT APPLICATION

(11) **EP 2 733 200 A1**
(43) Date of publication of application: **21.05.2014**
(21) Application number: 12192815.4
(22) Date of filing: 15.11.2012
(51) Int. Cl.: C12N 5/00

(54) **Cell culture supplements**

(71) Applicant: Biorigen International SA, 6900 Lugano (CH)
(72) Inventor: Muraglia, Anita, 17024 Finale Ligure (SV) (IT); Mastrogiacomo, Maddalena, 16132 Genova (IT); Cancedda, Ranieri, 16145 Genova (IT)
(74) Representative: Capasso, Olga

(57) **Abstract**

The invention relates to cell culture supplements based on a platelet rich plasma fraction and a platelet poor plasma fraction. The supplements increase cell proliferation rate, improve selection of clonogenic cells, proliferation of cells from biopsies from elderly patients, maintenance of cell differentiation potential, and in vitro expansion of cell cultures also starting from an extremely low number of initially plated cells. The supplements may be freeze-dried and kept for long period of time as a quality controlled "off the shelf" product.

## Description

### BACKGROUND OF THE INVENTION

Fetal bovine serum, or FBS or fetal calf serum, is the portion of plasma remaining after coagulation of blood, during which process the plasma protein fibrinogen is converted to fibrin and remains in the clot. FBS comes from the blood taken from a bovine fetus via a closed system of collection at the slaughterhouse.

FBS is the most widely used serum-supplement for the in vitro cell culture of eukaryotic cells. This is due to the fact that it has a very low level of antibodies and contains much growth factors. FBS is very versatile and can be used in many different cell culture applications. However, FBS as well as other products deriving from bovine source, are not advisable due to the risk of prion, zoonose and viral contaminations. In particular viral contamination concerns bovine spongiform encephalopathy, commonly known as "mad-cow disease", which is a fatal neurodegenerative disease in cattle that causes a spongy degeneration in the brain and spinal cord and, in humans, is known as new variant Creutzfeldt-Jakob disease. In addition, although FBS can support proliferation of many types of cells, it fails to promote proliferation of some human and animal cells, including articular chondrocytes from elderly subject donors. FBS also does not support certain culture conditions, such as some cell lines (Hela, etc.) or primary culture (skin fibroblasts, osteoblasts, etc.) plated at clonal densities.

Platelet Rich Plasma (PRP) is blood plasma that has been enriched with platelets. PRP contains several different growth factors and other cytokines that stimulate healing of bone and soft tissue.

Platelet Lysate (PL) is a component of PRP. It is obtained from PRP fraction of the blood by a freeze-thaw cycle repeated at least twice in order to break the platelet membranes and to release the growth factors content (platelet activation, activated PRP). The activated PRP is then centrifuged at high speed to precipitate the broken platelet membranes. Since PL does not contain red blood cells and related immunogenic factors (i.e. blood group-related antibodies), its applications present the benefit to avoid possible immunogenic reactions.

PRP and PL are used to enhance the proliferation of cell cultures, becoming therefore a substitute of FBS. Different articles in literature have reported that PRP and PL from human or animal (other than bovine) sources are effective and even more beneficial substitutes for fetal bovine serum to support *in vitro* expansion of human or animal cells (i.e. bone marrow stromal cells, mesenchymal stem cells) for different clinical and therapeutic applications (Zaky SH et al. J. Tissue Eng. Regen. Med. 2008, 2, 472-481; Castegnaro S et al. Curr. Stem Cell. Res. Ther. 2011, 6, 105-114; Schallmoser K et al. Transfusion 2007, 47, 1436-1446; Kocaöemer A et al. Stem Cells 2007, 25, 1270-1278; Bieback K et al. Stem Cells 2009, 27, 2331-2341; Aldén A et al. Cytotechnology 2007, 55, 3-8; Müller I et al. Cytotherapy 2006, 8, 437-444).

US patent application 2011200642 discloses compositions consisting of an agent which induces an inflammatory healing response combined with a platelet lysate from autologous source at a specific concentration which may have demonstrated *in vitro* abilities to expand autologous tissue repair cells.

US patent application 2011183414 describes a cell growth medium comprising (a) a human platelet lysate, (b) a human fresh frozen plasma, (c) heparin, (d) L-glutamine and (e) a serum-free, low glucose medium suitable for mammalian cell growth, wherein the cell growth medium permits the expansion of human CD34-stem cells and wherein the resulting expanded CD34-stem cells retain the ability to differentiate.

US patent application 2011171731 discloses methods and materials for using platelet lysate compositions to grow stem cells, to differentiate stem cells, to grow primary cell cultures, and to identify effective growth factors. The compositions containing platelet lysate are formulated with any appropriate medium to produce a culture medium having enhanced properties, such as DMEM, RPMI, AIMV, X-VIVO15 and other defined serum free or serum requiring media. The compositions containing platelet lysate are also formulated with one or more factors capable of differentiating cells, such as polypeptides, steroids, hormones, dexamethasone, EGF, FGF and BMP4.

US patent application 2010015710 concerns compositions for isolating and expanding human mesenchymal stem/progenitor cells through multiple passages in defined serum-free environments. The complex culture media compositions includes a basal medium supplemented with a nutrient mixture (Ham's F12, glutamine), buffer solutions (sodium bicarbonate), serum albumin, a lipid mixture, insulin, transferrin, putrescine, progesterone, fetuin, hydrocortisone, ascorbic acid or its analogues, growth factors, and platelet lysate as a serum-free substitute.

The international publication WO 2008/110570 discloses medium for culturing endothelial cells which comprises EGM-2, hydrocortisone, VEGF as well as human platelet lysate as supplement. US 5,198,357 describes platelet lysate used to entirely or partially replace fetal calf serum in cell culture. The platelet lysate is produced from animal plasma and contains an added citrate to prevent coagulation of the blood during storage. The lysate is prepared by centrifuging plasma to produce a platelet rich paste, adding calcium to the paste to lyse the platelets therein and coagulate fibrinogen to produce a clear liquid containing lysed platelets, sterile filtering the liquid and collecting a liquid filtrate containing the lysed platelets.

The international publication WO 2008/034803 concerns a platelet concentrate, such as platelet-rich plasma, used in a cell culture medium to grow and proliferate cells, which may be from autologous source. The cells grown in the culture medium may be used to treat a patient. The supplement further comprises albumin and/or dextran and/or hydroxyethyl starch.

The international publication WO 2007149328 discloses a defined serum-free cell culture media useful for culturing fibroblasts, especially dedifferentiated articular cartilage cells. The media avoid problems inherent to the use of serum-containing media. The defined media comprise platelet-derived growth factor (PDGF), chemically defined lipids, oncostatin M, interleukin-6 (IL-6), leukemia inhibitory factor, or combinations of these compounds.

US patent application 2010120144 describes a concentrated blood component, such as platelet-rich plasma, used in a cell culture medium to grow and proliferate cells to treat a patient, possibly with cells from autologous source.

US patent application 2009305401 provides a cell culture medium supplement comprising plasma-free platelet lysate prepared by lysing the platelets from platelet rich plasma. The medium is supplemented with albumin, dextrane, or hydroxyethyl starch.

JP 8308561 discloses the preparation of a medium for culturing cells without adding a serum and without losing functionality of the cells. The medium is composed of a water-soluble selenium added to a mixture of cell stimulating compounds, among them platelet-derived growth factor (PDGF).

All cited documents deal with compositions aimed to improve, enhance, tune the quality and yield for isolation, proliferation, expansion and differentiation of cell cultures.

Nevertheless, experimental data show that platelet derived products used as cell culture supplements may have different effects depending on the starting platelet concentration and on the preparation procedure. Despite the extensive literature about the use of PRP and PL as efficient substitutes of animal serum (Pawitan JA, Curr Stem Cell Res Ther. 2012, 7, 329-35; Schallmoser K et al. J Vis Exp. 2009, 30; pii: 1523, doi: 10.3791/1523; Anitua E et al. Cell Prolif. 2009, 42, 162-70; Kinzebach S et al. Adv Biochem Eng Biotechnol. 2012; Blande IS et al. Transfusion. 2009, 49, 2680-5; Ben Azouna N et al. Stem Cell Res Ther. 2012, 3, 6; Lohmann M et al. PLoS One. 2012, 7, e37839; Weibrich G et al. J Craniomaxillofac Surg. 2002; 30, 97-102; Lange C et al. J Cell Physiol. 2007, 213, 18-26; Doucet C et al. J Cell Physiol 2005, 205, 228-36: Gruber R et al. Platelets 2004, 15, 29-35) there is still a lack of standardized protocols for platelet derived products preparation.

In fact, since the platelet concentration is highly variable from individual to individual and for the same individual it can vary from time to time, the "traditional" platelet derived preparations such as PRP and PL used as culture medium supplements do not allow the reproducibility and the consistency of the experimental results. In particular this is due to the fact that PRP and PL contain undefined concentrations of factors and molecule. Moreover, there is still the need for a medium supplement that is practical and of easy handling.

### DESCRIPTION OF INVENTION

The authors of the present invention have surprisingly found that cell culture supplement formulations based on two different platelet and plasma derived components, optionally containing defined concentrations of a specific anti-coagulant agent, allow the preparation of cell culture medium additives that may be optimized for any specific cell type.

The authors of the present invention have used the different components as above described. The components are combined in different ratios and are used as serum-supplement(s) for *in vitro* cell culture to substitute Fetal Calf Serum (FBS).

Indeed, the authors' platelet derived products cannot be merely only regarded as additives for cell culture media to replace FBS or other animal sera. In particular, the authors surprisingly found that each type of cell requires a specific ratio between the content of platelet derived factors and bioactive molecules and plasma (or serum). The formulations are based on two components, combined in different ratios and optionally containing a defined concentration of a specific anti-coagulant agent. The first component is a derivative obtained starting from a platelet/plasma fraction containing a well-defined number of platelets per volume and the second component is a derivative from platelet poor plasma.

The first component, or Component A, is a substantially platelet rich plasma fraction. The Component A may be optionally processed in order to remove the fibrinogen part (serum) to avoid the formation of fibrin rich clot once activated (the fibrinogen-free component A is named Component C). When Component C is used, there is no need to the addition of an anti-coagulant agent.

The second component, or Component B, is a derivative from platelet poor plasma. The Component B may be optionally processed in order to remove the fibrinogen part (the fibrinogen-free component B is named Component D). When component D is used there is no need for the addition of an anti-coagulant agent. The platelet poor plasma is blood plasma with very low number of platelets (< 50 x 10³/µL). When using the mixture of Component A and Component B, an anti-coagulant agent is usually used in an optimal ratio. Because fibrinogen may be present in the supplement solution, the anti-coagulant agent prevents the conversion of fibrinogen into fibrin and, therefore allows to carry out an easier and cleaner process. In fact, the conversion of fibrinogen into fibrin involves the formation of agglomerates which can interfere with the cell culture by limiting the cell growth and differentiation and/or by making a more difficult operative handling of the culture steps.

The amount (concentration) of the two components in the mixture and used in cell cultures is, in any case, lower than that of FCS to obtain the same performances. The supplement of the invention shows improved performances.

The mixture of the two components (Component A + Component B or Component C + Component D) at an optimal ratio, with optionally the addition of an anti-coagulant agent at an optimal ratio with the mixture, increased cell proliferation rate (cell doubling time) in both primary cells and cell lines.

Moreover, the same mixtures improve selection of clonogenic cells and/or cell sub-populations. They allow proliferation of cells from biopsies obtained from elderly patients, otherwise not possible with FCS. Further the mixtures maintain cell differentiation potential in human articular chondrocytes and mesenchymal stem cells. Moreover the mixtures allow an *in vitro* expansion of cell cultures starting from an extremely low number of initially plated cells.

Finally, the addition of the poor platelet plasma component (Component B and D) also enhances the cell culture dish coating and cell adhesion, as a further advantageous property.

The possibility of freeze drying the single components or their mixtures, of sterilizing them and of storing them for long period of time at low temperatures, allows the use of quality controlled "off the shelf" products.

The single component or mixtures thereof are preferably sterilized by gamma radiation or by filtration. The sterilization step may be performed either before or after freeze drying.

In summary, the platelet derived cell culture supplement of the present invention are based on the combination, in different ratios, of two different plasma and platelet derived components processed from blood platelet enriched fractions with a defined and optimized number of platelets per volume. The present invention provides the preparation and use of cell culture supplements specifically optimized for each cell type together with improved reproducibility of the cell culture conditions.

It is therefore an object of the present invention a cell or tissue culture medium supplement comprising:
a) from 95 % to 0.5 % (volume/volume) of a platelet rich plasma fraction containing at least 2x10⁶ platelets/mL;
b) from 5 % to 99.5 % (volume/volume) of a platelet poor plasma fraction containing less than 5x10⁴ platelets/mL and
c) optionally an anti-coagulant agent.

Preferably the cell culture medium supplement comprises:
- from 70 % to 2.5 % (volume/volume) of the platelet rich plasma fraction and
- from 30 % to 97.5 % (volume/volume) of the platelet poor plasma fraction.

Still preferably the cell culture medium supplement comprises:
- from 60 % to 5 % (volume/volume) of the platelet rich plasma fraction and
- from 40 % to 95 % (volume/volume) of the platelet poor plasma fraction.

Yet preferably, the cell culture medium supplement comprises 75% of the platelet rich plasma fraction and 25% (volume/volume) of the platelet poor plasma fraction.

Yet preferably, the cell culture medium supplement comprises 95% of the platelet rich plasma fraction and 5% (volume/volume) of the platelet poor plasma fraction.

In a preferred embodiment the platelet rich plasma fraction contains from 5x10⁶ to 15x10⁶ platelets/mL, preferably from 8x10⁶ to 12x10⁶ platelets/mL.

In a still preferred embodiment the platelet poor plasma fraction contains less than 1x10⁴ platelets/mL, preferably less than 0.4x10⁴ platelets/mL

In a yet preferred embodiment the platelet rich plasma fraction is further subjected to a lysis step and a centrifugation step.

Preferably the platelet rich plasma fraction and/or the platelet poor plasma fraction is processed to remove fibrinogen.

Still preferably the anti-coagulant agent is heparin.

Heparin is a well-known and widely used anticoagulant, preventing the formation of clots and extension of existing clots within the blood through a body's natural clot lysis mechanisms (Tahir RA, US Pharm. 2007, 32, HS26-HS36; Hirsh J et al. Chest 2001, 119, 64S-94S).

The role of heparin when added to cell cultures is still largely unknown and controversial. Heparin has shown both anti-proliferative action in tumor cell lines, thus evidencing a toxic behavior towards cells (Abu Arab W et al. Can J Physiol Pharmacol. 2011, 89, 705-711; Ichikawa J et al. Cancer 2012, 118, 2494-256) and amplification of growth factors activity and proliferation of stem cells (Hudalla GA et al. Adv Mater. 2011, 23, 5415-5418; Frescaline G et al. Stem Cell Res. 2012, 8, 180-192).

In the proposed range with the cell culture supplement of the invention, it is pointed out that heparin explicates its well-known action as anti-coagulant agent, thus allowing an easier and cleaner manipulation of the cell culture, and also enhance the proliferation of cells in culture.

In a still preferred embodiment the concentration of anti-coagulant in the supplement ranges from 40U/ml to 4,000U/ml.

The final concentration of anti-coagulant in the cell culture medium (when 5 % of supplement is used) ranges from 2 U/ml to 200U/ml, preferably from 4U/ml to 100U/ml, most preferably from 10U/ml to 30U/ml.

In a still preferred embodiment the cell culture medium supplement is frozen and/or freeze-dried and/or sterilized. Preferably the sterilization is performed before or after the freeze-drying step. Still preferably the sterilization is performed by gamma radiation or filtration.

It is a further object of the invention a process for the preparation of the cell culture medium supplement according to any one of previous claims comprising mixing the platelet rich plasma fraction, the platelet poor plasma fraction and the anti-coagulant agent wherein the platelet rich plasma fraction and/or the platelet poor plasma fraction and/or the anti-coagulant agent are in liquid or powder form.

It is a further object of the invention a method to *in vitro* expand a cell and /or to promote proliferation and/or differentiation of a cell and/or select of clonogenic cell and/or a cell sub-population and/or to maintain the differentiation potential of a cell comprising culturing said cell in a medium supplemented with 0.1 to 30 % of the cell culture medium supplement as defined above.

Preferably, the cell is cultured in a medium supplemented with 0.5 to 20 % of the cell culture medium supplement as defined above. Most preferably the cell is cultured in a medium supplemented with from 1% to 15% of the cell culture medium supplement as defined above. Still preferably the cell is selected from the group consisting of: a primary cell, a cell line, a cell obtained from a biopsy of an elderly patient, an articular chondrocyte, a stem cell and an iPS cell.

In a preferred embodiment the cell is plated at density below 3 X 10³ per cm².

According to the invention, the first component which contains a well-defined and optimized number of platelets per volume, is, as a not limitative example, platelet rich plasma, alone or suitably pre-diluted with a small fraction of platelet poor plasma, and platelet lysate, obtained by lysis of platelet rich plasma, alone or suitably pre-diluted with a small fraction of platelet poor plasma to get the desired concentration range.

According to the invention, the first component can be derived from plasma or is suitably derived after processing to eliminate the fraction which can trigger coagulation.

According to the invention, the first component is mixed with the second component, preferably by combining Component A (fraction with higher platelet concentration) and Component B (platelet poor fraction), both with fibrinogen and agents triggering coagulation, or by combining Component C (fraction with higher platelet concentration) and Component D (platelet poor fraction), both without fibrinogen and agents triggering coagulation.

According to the invention, the mixture containing the first and second component, optionally with the addition of anti-coagulant agent, is used for primary cell cultures and cell lines.

According to the invention, the mixture containing the first and second component, optionally with the addition of anti-coagulant agent is used, as a not limitative example, for cell proliferation and differentiation, selection of clonogenic cells and/or cell sub-populations, proliferation of cells from biopsies obtained from elderly patients, maintaining the cell differentiation potential as, for example, in human articular chiondrocytes and mesenchymal stem cells, in vitro expansion of cell cultures also starting from an extremely low number of initially plated cells.

According to the invention, the components and/or their mixtures are frozen and freeze-dried for a long term storage at low temperature.

The possibility of freeze drying the mixture preparations, sterilizing (either before or after freeze drying) and storing them for long time at low temperatures, allows therefore the usage of quality controlled "off the shelf" and "ready to use" products.

According to the invention, the components and/or their mixtures are sterilized, preferably by gamma radiation, before or after freeze-drying process, or by filtration.

The invention will be now described by reference to the following non-limiting examples.

### EXAMPLES

### Example 1

### Preparation of Component A, - platelet rich plasma fraction (PRP)

Component A is the fraction with an optimal concentration of platelet.

Component A is prepared starting from human blood buffy coats (Hospital Transfusional Center of Genova). Buffy coat samples from 10 to 20 healthy donors are pooled together in a single sterile blood bag connected to a satellite blood bag. The pooled buffy coat containing bag is centrifuged at low speed (1,100 RPM x 10 minutes, centrifuge ROTOSILENTA 630 RS, Hettich) and the upper phase represented by the platelet rich plasma fraction (PRP) is collected in the satellite bag, while the red blood cells containing phase is discarded. The platelet concentration in the PRP is measured by means of an automatic hemocytometer.

The PRP containing bag is connected in a sterile way to a satellite bag and subjected to a second high speed (2,600 RPM X 20 minutes, centrifuge ROTOSILENTA 630 RS, Hettich) centrifugation. After this second centrifugation, the platelets are concentrated at the bottom of the bag and the upper phase, represented by the platelet poor plasma fraction (PPP, Component B) is transferred to the satellite empty bag.

After measuring the platelet concentration of PRP, PRP can optionally be re-suspended in a known PPP volume in order to obtain a final platelet concentration final platelet concentration from 8x10⁶ to 12x10⁶ platelets/µl.

The bag containing the plasma derivative at a desired concentration is then transferred to a - 80°C freezer or freeze-dried and stored at -20°C.

### Example 2

### Preparation of Component A - Platelet Lysate (PL)

The PRP fraction prepared as in Example 1 and kept at -80°C at least for 15 hours is gently thawed at 37°C and the platelet extract transferred to a bag which is frozen down to liquid nitrogen temperature (-196°C). Then the bag is thawed at 37°C. This freeze-thaw cycle is repeated for three times in order to fully break the platelets membranes and to obtain a Platelet Lysate (PL). After the third cycle is completed the bag is centrifuged at high speed (4,500 RPM X 20 minutes, centrifuge ROTANTA 460R, Hettich) to remove platelet membranes and cell debris. The clear liquid fraction within the bag is carefully separated from the settled membranes and debris and collected in a sterile container under a laminar flow hood.

The liquid is then frozen at -80°C, freeze-dried, sterilized by gamma radiation (1 KGy) and stored at -20°C.

### Example 3

### Preparation of Component B, platelet poor plasma (PPP) fraction

Component B is obtained during Component A preparation in the procedure described in Example 1. (number of platelets < 50 x 10³/µL). Component B is freeze-dried, sterilized by gamma radiation (1 KGy) and then stored at -20°C.

Before the freeze-drying process, different concentrations 4 to 100 U/ml of heparin can be added to the Component B.

### Example 4

### Preparation of Component C

Component C is a Component A without fibrinogen and fraction of coagulating agents.

Component C is prepared starting from pooled buffy coats samples. Up to the 3 freeze-thaw cycles the preparation protocol followed is the same described for Component A ESEMPIO 2, At the end of the last freeze-thaw cycle, the platelet extract is mixed with 0.1 M CaCl₂ (9:1 volume:volume) to trigger a clot formation. Alternatively at the end of the last freeze-thaw cycle, the bag containing the platelet extract is centrifuged at 4,500 RPM X 20 minutes (centrifuge ROTANTA 460R, Hettich) to remove membranes and cell debris and the supernatant collected in another bag prior the mixing with the 0.1M CaCl₂ solution. Bags are then left for 12-20 hours at room temperature in constant linear agitation to facilitate the clot formation. The bag is then centrifuged at high speed (4,500 RPM for 20 minutes, centrifuge ROTANTA 460R, Hettich) to sediment the formed clot. The liquid fraction is collected in a tube and further centrifuged at 3150 RPM for 10 minutes (centrifuge 5810R, Eppendorf) and the liquid clear phase is collected in sterile tubes under a laminar flow hood. The tubes are then frozen at -80°C, freeze-dried and sterilized by gamma radiation (1 KGy). Alternatively to the gamma irradiation, the final liquid preparation could be sterilized by filtration (0.45 - 0.22 microns) prior to freeze-thawing.

### Example 5

### Preparation of Component D

Component D is a Component B without fibrinogen and fraction of coagulating agents.

The PPP residual from the Component A PRP preparations (Example 1) contained within the blood bag is mixed with 0.1M CaCl₂ (9:1 volume:volume) and the bag is left for about 4 h hours at room temperature in constant linear agitation to obtain the maximum of the clot formation. CaCl₂ causes coagulation of the plasma. The bag is then centrifuged at high speed (4,500 RPM X 20 minutes, centrifuge ROTANTA 460R, Hettich) to remove the clot. The liquid fraction is collected in a tube and further centrifuged at 3150 RPM for 10 min (centrifuge 5810R, Eppendorf) and the liquid clear phase collected in sterile tubes under a laminar flow hood. The product is freeze-dried, sterilized by gamma radiation and stored as described above for Component C. Alternatively to gamma irradiation, (1 KGy). the final liquid preparation may be sterilized by filtration (0.45 - 0.22 microns) prior to freeze-thawing.

### Example 6

### Proliferation rate (MTT assay)

Primary cultures of human osteoblasts (hOB), human bone marrow stromal cells (hBMSC), human skin fibroblasts (hSF) and human articular chondrocytes (hAC) were established from tissue obtained during surgery and otherwise destroyed. All procedures were performed after obtaining informed consent from patients and Ethical Committee approval. Cells have been initially plated at 4 to 8 x 10³ per 1 cm² and cultured with basal tissue culture medium (Iscove's modifies Dulbecco's medium Euroclone ECM0192L; Coon's modified Ham's F12, Biochrom cod. FZ0855; D-MEM Euroclone ECB7501L) supplemented with 10% FCS and incubated at 37°C in humidified atmosphere containing 95% air and 5% CO₂. At confluence cells were detached and replated at low density in each well of a 24-well plate. The day after, cells (6 x 10³ per 1 cm²) were transferred to serum-free medium supplemented with 5% of a mixture of Component A (75%) and Component B (25%).

The cultures were tested for proliferation rate with the MTT assay (Sigma, M5655), which measures cellular metabolic activity and may, under defined conditions, reflect the number of viable cells (cell proliferation). Cells grown in 10% FCS were used as control condition.

The results are indicated in Table 1.

**Table 1. MTT assay (expressed as an optical density between 570 and 670 nm) after 200 hours of cultures containing 5% of a mixture of Component A (75%) and Component B (25%) or of cultures containing 10% FCS.**

| Primary culture | OD (200 h) 5% Mixture | OD (200 h) 10% FCS |
|---|---|---|
| hOB | 1,40 | 0,14 |
| hBMSC | 0,65 | 0,36 |
| hSF | 0,60 | 0,22 |
| hAC | 1,17 | 0,18 |

The results show that the mixture (at half the concentration of FCS) has a higher mitogenic effect on the analyzed cell culture populations when compared to the FCS control.

### Example 7

### Proliferation rate with platelet Lysate

Primary cultures of articular chondrocytes (hAC) have been plated at 1 X 10⁵ per 6 cm diameter dish in a serum-free medium (Coon's modified Ham's F12, Biochrom cod. FZ0855) supplemented with 5% of a mixture of Platelet Lysate (prepared from Component A, example 2) (75%) and Component B (25%). The cultures were tested for proliferation rate with the determination of doubling number that occurs per unit of time (after 30 days). A 5% mixture of Component A (75%) and Component B (25%) was used as control condition. The results are indicated in Table 2.

**Table 2. Doubling number after 30 days of cultures with mixtures containing Component A and Component A as a platelet lysate (PL).**

| 5% mixture of Component A (75%) and Component B (25%) | 5% mixture of Platelet Lysate (prepared from Component A, example 2) (75%) and Component B (25%) |
|---|---|
| 23 | 22 |

No significant difference between the two used mixtures (with and without platelet lysate as a Component A) was observed.

### Example 8

### Proliferation rate (doubling number)

Primary cultures of articular chondrocytes from articular cartilage biopsy have been cultured with basal tissue culture medium (Coon's modified Ham's F12, Biochrom cod. FZ0855) supplemented with 5% of a mixture of Platelet Lysate (prepared from Component A, example 2) (75%) and Component B (25%) or supplemented with a 5% of a mixture of Component C (75%) and Component D (25%). Cells were incubated at 37°C in humidified atmosphere containing 95% air and 5% CO₂. At first confluence, cells were detached and replated at low density 1 x 10⁵ in 6 cm diameter dish and tested for proliferation rate with the determination of doubling number that occurs per unit of time (after 30 days). Cells grown in 10% FCS were used as control condition. The results are presented in Table 3.

**Table 3. Doubling number after 30 days of cultures containing different concentrations of mixture (10% FCS, doubling number = 2).**

| % in cell culture | Platelet Lysate (prepared from Component A, example 2) (75%) and Component B (25%) | Component C (75%) and Component D (25%) |
|---|---|---|
| 0,5 | 5 | 6 |
| 1 | 10 | 10 |
| 2,5 | 19 | 18 |
| 5 | 22 | 23 |
| 10 | 18 | 18 |
| 20 | 3 | 4 |

The results show that both mixtures enhance cell population growth rate when compared with 10% FCS control (doubling number after 30 days = 2). This effect is observed even at a very low concentration of the mixtures. No significant differences were observed between the two mixtures.

### Example 9

### Addition of heparin

Human umbilical cord blood derived cells from donned cord have been cultured with basal tissue culture medium (alpha-MEM Glutamax, GIBCO 32561) supplemented with 5% of a mixture of Platelet Lysate (prepared from Component A, example 2) (50%) and Component B (50%) further containing different concentrations of heparin. Cell proliferation was evaluated via MTT assay (Sigma M5655). The results are reported in Table 4.

**Table 4. MTT assay (expressed as an optical density between 570 and 670 nm) after 120 hours of cultures containing increasing concentration of heparin in a 5% of Platelet Lysate (prepared from Component A, example 2) (50%) and Component B (50%).**

| Heparin concentration (U/mL) | OD (570-670 nm) |
|---|---|
| No heparin | - |
| 0,5 | - |
| 1 | - |
| 2 | 1.25 |
| 10 | 1.32 |
| 20 | 1.20 |
| 50 | 0.83 |

The results show that an optimal range (2-20 U/mL) of heparin enhance the proliferation rate when compared to other heparin concentrations.

### Example 10

### Clonogenicy (hBMSC)

The clonogenic potential of hBMSC was evaluated with the colony forming unit-fibroblast assay (CFU-f). Bone marrow nucleated cells were plated at 7.5 to 12.5 X 10³ per cm² in basal tissue culture medium (Coon's modified Ham's F12, Biochrom cod. FZ0855) supplemented with 5% of a mixture of Platelet Lysate (prepared from Component A, example 2) (75%) and Component B (25%) or in the same medium supplemented with 10% FCS or 10% FCS + bFGF (1 ng/ml, Perprotech, 100-18b) (control conditions).

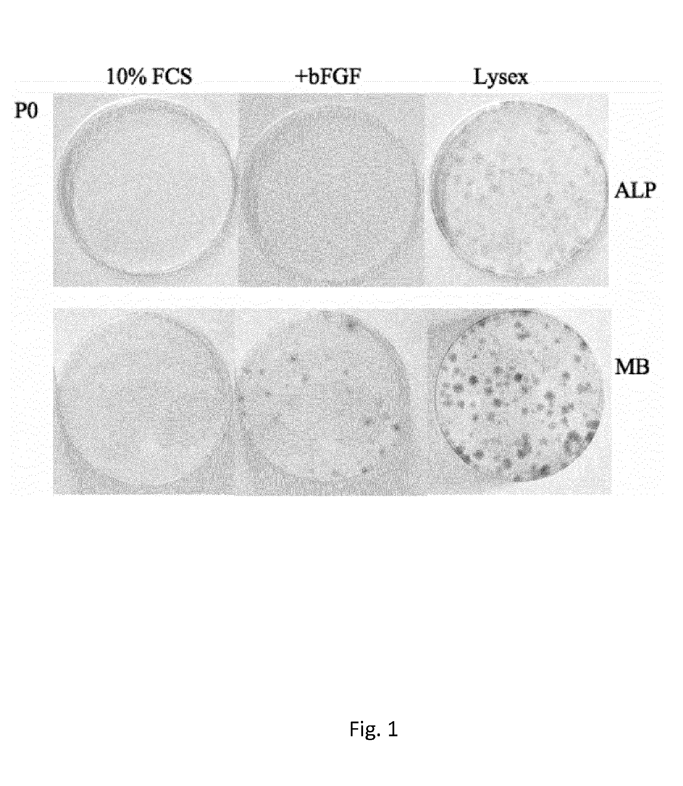

After 14 days of culture, colonies were stained for alkaline phosphatase expression (ALP) and methylene blue (MB). The results are presented in Fig. 1. Fig. 1 shows that the 5% mixture of Platelet Lysate (prepared from Component A, example 2) (75%) and Component B (25%) (named "Lysex") increases the total number of colonies and the number of ALP positive colonies (90-100%) when compared to both 10% FBS and 10% FCS+ bFGF .

### Example 11

### Proliferation of cells derived from elderly patients

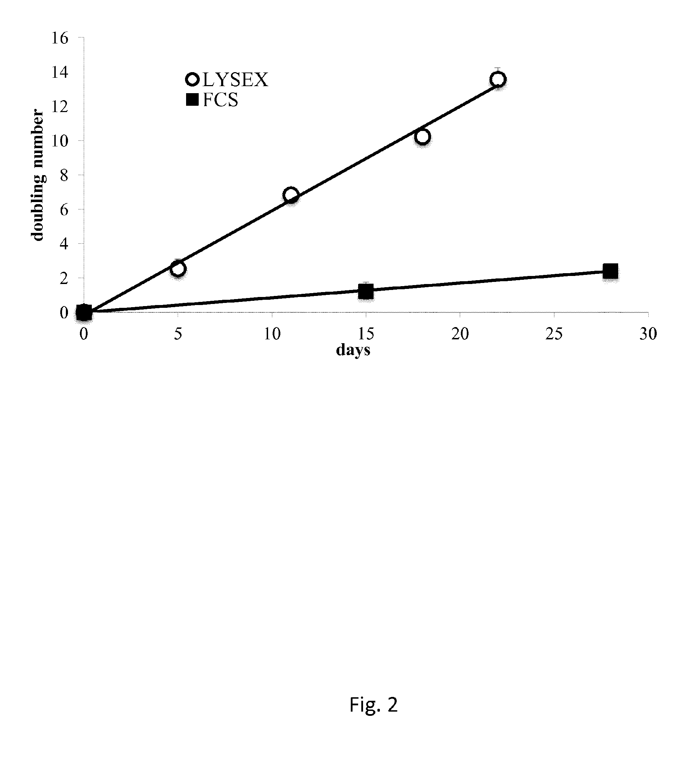

Human articular chondrocytes (hAC) from articular cartilage biopsy have been cultured with basal tissue culture medium (Coon's modified Ham's F12, Biochrom cod. FZ0855) supplemented with 5% of a mixture of Platelet Lysate (prepared from Component A, example 2) (75%) and Component B (25%) (named "Lysex") or in the same medium supplemented with 10% FCS (FCS, control). The cell population doublings were evaluated at different time points as shown in Fig. 2. Values represent the number of doublings obtained at 80% confluence in the culture time period range (n = 3 primary cultures).

The results demonstrate that hAC cells expanded in a medium supplemented with 5% of a mixture of Platelet Lysate (prepared from Component A, example 2) (75%) and Component B (25%) (named "Lysex") showed a higher proliferation potential when compared to hAC cells expanded in 10% FCS (Fig. 2).

### Example 12

### hAC micromass assay

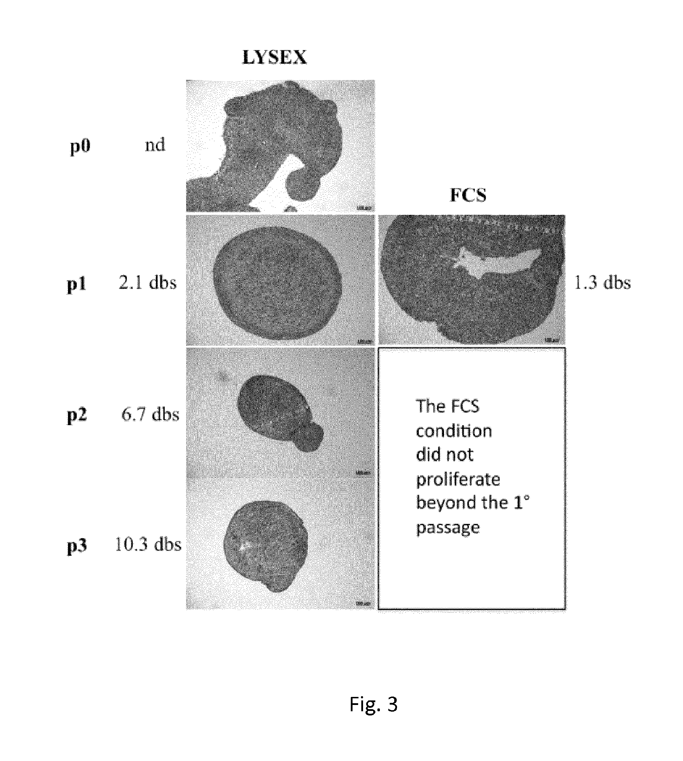

Human articular chondrocytes from articular cartilage biopsy have been expanded in basal tissue culture medium (Coon's modified Ham's F12, Biochrom cod. FZ0855) supplemented with 5% of a mixture of Platelet Lysate (prepared from Component A, example 2) (75%) and Component B (25%) (named "Lysex") or in the same medium supplemented with 10% FCS. Cells were tested for *in vitro* differentiation in the micromass culture assay (Johnstone B. et al. 1998, Exp. Cell Res. 238,265-272). The results are shown in Fig. 3.

Cells expanded in the medium supplemented with 5% of a mixture of Platelet Lysate (prepared from Component A, example 2) (75%) and Component B (25%) (named "Lysex") undergo chondrogenic differentiation, comparable to FCS expanded cells, and produce a metachromatic cartilaginous matrix (toluidine blue staining) incorporating chondrocytes within lacunae (Fig. 3). In Fig. 3, human articular chondrocytes were expanded in 5% LYSEX (A) or 10% FCS (B) and tested for in vitro differentiation in the micromass culture assay at different passages in culture (p0, p1, p2, etc.). Each passage in culture corresponds to a different doubling number. Cells expanded in 5% LYSEX undergo a higher doubling number (dbs) in comparison with 10% FCS expanded cells which do not proliferate beyond the first passage (p1).

Cells derived from the expansion in LYSEX supplemented medium undergo chondrogenic differentiation as well as FCS expanded cells producing a methacromatic cartilagineous matrix (toluidine blue staining) with chondrocytes in lacunae.

This finding provides evidence of the maintenance of the chondrogenic potential by the chondrocytes expanded in the presence of the platelet product. It is to note that the chondrogenic differentiation is maintained also after a number of doublings never reached by chondrocytes expanded in the presence of FCS.

### Example 13

### hBMSC

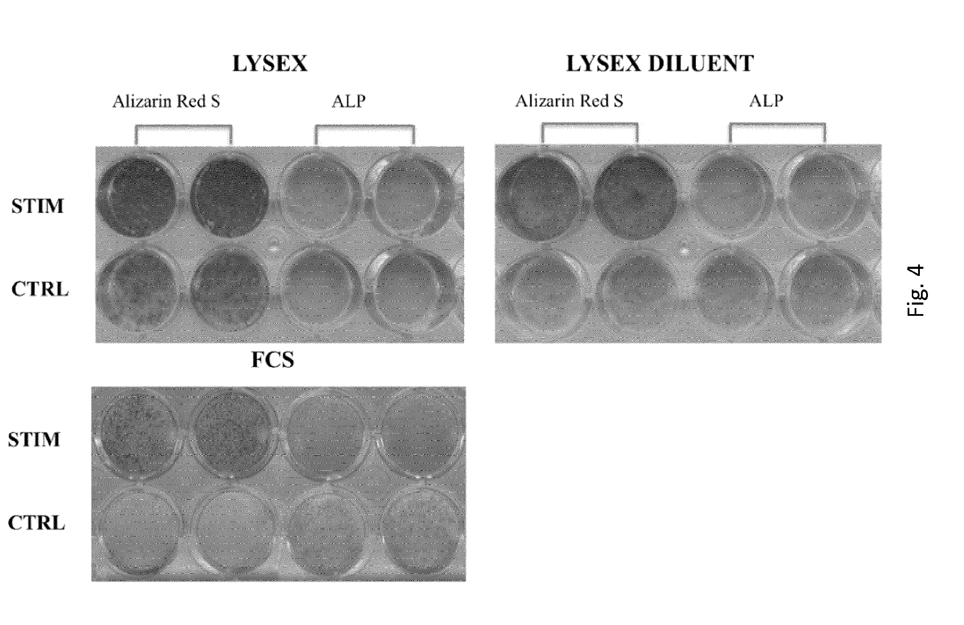

hBMSC from iliac crest aspirates have been expanded in a basal tissue culture medium (Coon's modified Ham's F12, Biochrom cod. FZ0855) supplemented with 5% of a mixture of Platelet Lysate (prepared from Component A, example 2) and Component B (3:1 volume:volume) (named "Lysex" in Fig. 4) or with 5% of only Component B (named "Lysex diluent" in Fig. 4) or with 10% FCS containing medium (control condition, named FCS in Fig.4). At confluence, cells have been induced with an osteogenic medium (Muraglia A. et al. J. Cell Sci. 2000, 113, 1161-1166) containing ascorbic acid (50 µg/ml), dexamethasone 10⁻⁷ M) and β glycerophosphate (10 mM) every other day (STIM). The *in vitro* osteogenic differentiation was assessed by means of histochemical staining with Alizarin Red S which stains in red the mineralized matrix and by Alkaline Phosphatase (AP) staining which stains in violet cells positive for the AP osteogenic marker. The results are presented in Fig. 4

Fig. 4 shows that cells expanded in the medium supplemented with 5% of a mixture of Platelet Lysate (prepared from Component A, example 2) and Component B (3:1 volume:volume) and cells expanded in the medium supplemented with Component B differentiated earlier (10 days compared to 15-20 days for FCS) than cells expanded with FCS (weak staining). Further, those cells were able to produce a marked osteogenic mineralized matrix.

### Example 14

### Cell density plating

Hela cells were obtained from the cell bank of the authors' institute (www.iclc.it) and their proliferation in the different media was evaluated by means of the xCELLIgence technology (RTCA DP Instrument, Roche Applied Science) which evaluates the "cell index" parameter. Cells were cultured with 5% of a mixture of Platelet Lysate (prepared from Component A, example 2) and Component B in different relative ratios and 5% FCS (control). The cells were plated at different densities (0.5 to 4 X 10³ per well and, as indicated in Table 5.

**Table 5. Cell index after 120 hours of culture in presence of 5% of a mixture of Platelet Lysate (prepared from Component A, example 2) and Component B, at different relative ratios between the Components, or 5% FCS in plates at different cell densities.**

| 5% supplemented medium | | Cell density (cells/well) | | |
|---|---|---|---|---|
| | | 500 | 2000 | 4000 |
| FCS | | 0,22 | 1,4 | 1,5 |
| Platelet Lysate (prepared from Component A, example 2) (%) | Component B (%) | | | |
| 0 | 100 | 0,51 | 0,98 | 1,33 |
| 10 | 90 | 1,66 | 1,82 | 2,23 |
| 20 | 80 | 1,22 | 2,03 | 2,21 |
| 50 | 50 | 1,08 | 1,70 | 2,00 |
| 70 | 30 | 1,00 | 1,31 | 1,60 |
| 100 | 0 | 0,42 | 0,81 | 1,30 |

The results show that an optimal range of 10 to 20% of Platelet Lysate (prepared from Component A, example 2) induces the best cell proliferation. Moreover, the proliferation was observed also when the cells were plated at very low concentration at variance with the behavior of the same cells in control cultures when the supplement was 5-10% FCS (no cell growth at 500 cells per well (not shown).

### Example 15

### Stability

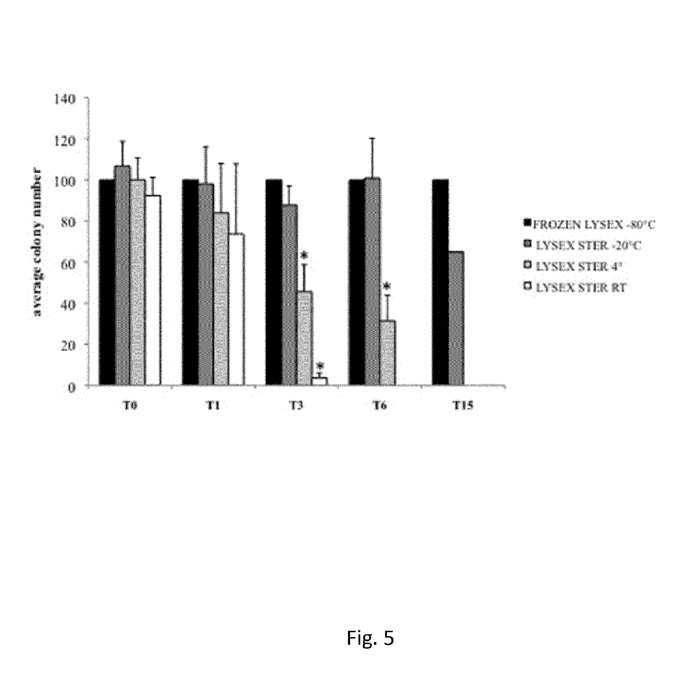

Different aliquots of a freeze-dried preparation, namely 75% Platelet Lysate (prepared from Component A, example 2) and 25% Component B ("Lysex"), have been stored at Room Temperature (LYSEX STER RT), 4°C (LYSEX STER 4°), -20°C (LYSEX STER -20°C). The frozen not freeze-dried lysex preparation stored at -80°C (FROZEN LYSEX -80°C) was used as internal control of the experiments. For each of these conditions, preparations have been evaluated in the clonogenic assay with hBMSC as decribed in Example 10 immediately after preparation (T0), 1 month (T1), 3 months (T3), 6 months (T6), 15 months (T15) after storage. The results are shown in Fig. 5. The colony number of cells cultivated in the presence of preparations stored at RT and 4°C significant decreased after 3 and 6 months of storage, respectively (Fig. 5). After 15 months of storage, the preparations stored at -20°C are still able to support colonies formation with an efficiency close to that at T0.

## Claims

1. A cell or tissue culture medium supplement comprising:
a) from 95 % to 0.5 % (volume/volume) of a platelet rich plasma fraction containing at least 2x10⁶ platelets/mL;
b) from 5 % to 99.5 % (volume/volume) of a platelet poor plasma fraction containing less than 5x10⁴ platelets/mL and
c) optionally an anti-coagulant agent.

2. The cell culture medium supplement according to claim 1 comprising:
- from 70 % to 2.5 % (volume/volume) of the platelet rich plasma fraction and
- from 30 % to 97.5 % (volume/volume) of the platelet poor plasma fraction.

3. The cell culture medium supplement according to claim 1 or 2 wherein the platelet rich plasma fraction contains from 5x10⁶ to 15x10⁶ platelets/mL.

4. The cell culture medium supplement according to any one of previous claims wherein the platelet poor plasma fraction contains less than 1x10⁴ platelets/mL.

5. The cell culture medium supplement according to any one of previous claim wherein the platelet rich plasma fraction is further subjected to a lysis step and a centrifugation step.

6. The cell culture medium supplement according to any one of previous claims wherein the platelet rich plasma fraction and/or the platelet poor plasma fraction is processed to remove fibrinogen.

7. The cell culture medium supplement according to any one of previous claims wherein the anti-coagulant agent is heparin.

8. The cell culture medium supplement according to any one of previous claims wherein the concentration of anti-coagulant in the supplement ranges from 40U/ml to 4,000U/ml.

9. The cell culture medium supplement according to any one of previous claim being frozen and/or freeze-dried and/or sterilized.

10. A process for the preparation of the cell culture medium supplement according to any one of previous claims comprising mixing the platelet rich plasma fraction, the platelet poor plasma fraction and the anti-coagulant agent wherein the platelet rich plasma fraction and/or the platelet poor plasma fraction and/or the anti-coagulant agent are in liquid or powder form.

11. A method to *in vitro* expand a cell and /or to promote proliferation and/or differentiation of a cell and/or select of clonogenic cell and/or a cell sub-population and/or to maintain the differentiation potential of a cell comprising culturing said cell in a medium supplemented with 0.1 to 30 % of the cell culture medium supplement as defined in claims 1 to 9.

12. The method of claim 11 wherein the cell is cultured in a medium supplemented with 0.5% to 20 % of the cell culture medium supplement as defined in claims 1 to 9.

13. The method according to claim 11 or 12 wherein the cell is selected from the group consisting of: a primary cell, a cell line, a cell obtained from a biopsy of an elderly patient, an articular chondrocyte, a stem cell and an iPS cell.

14. The method according to any one of claim 11 to 13 wherein the cell is plated at density below 3 x 10³ per cm².
